**Europäisches Patentamt**

**European Patent Office**

**Office européen des brevets**

(19)

(11) **EP 1 338 286 A1**

(12) **EUROPÄISCHE PATENTANMELDUNG**

(43) Veröffentlichungstag:
27.08.2003 Patentblatt 2003/35

(51) Int Cl.⁷: **A61K 35/78**, A61L 2/00,
A61P 31/12, A61P 31/04

(21) Anmeldenummer: 03003788.1

(22) Anmeldetag: **19.02.2003**

(84) Benannte Vertragsstaaten:
**AT BE BG CH CY CZ DE DK EE ES FI FR GB GR HU IE IT LI LU MC NL PT SE SI SK TR**
Benannte Erstreckungsstaaten:
**AL LT LV MK RO**

(30) Priorität: **20.02.2002 DE 10207174**

(71) Anmelder: **Dötsch-Jutsch, Christel, Dr.**
**19069 Alt Meteln (DE)**

(72) Erfinder:
- **Dötsch-Jutsch Christel**
**19069 Alt Meteln (DE)**
- **Mentel Renate**
**17493 Greifswald (DE)**
- **Lindequist Ulrike**
**17491 Greifswald (DE)**
- **Jülich Wolf-Dieter**
**17489 Greifswald (DE)**
- **Mundt Sabine**
**18439 Stralsund (DE)**

(54) **Verwendung von Extrakten aus Pflanzen der Familie Zosteraceae zur Vorbeugung und Therapie von bakteriellen und viralen Infektionen**

(57) Die Erfindung betrifft Extrakte aus Pflanzen der Familie der Seegrasgewächse, Zosteraceae, die antibakterielle und antivirale Eigenschaften aufweisen, und die spezielle human- und veterinärmedizinische Anwendungen insbesondere auf Haut und Schleimhäuten ermöglichen. Besonders vorteilhaft ist die Prophylaxe und Therapie von Herpesinfektionen sowie die Euterbehandlung zur Verhinderung von Staphylokokkenübertragungen in der Veterinärmedizin.

EP 1 338 286 A1

**Beschreibung**

**Stand der Technik**

Marine Blütenpflanzen zeichnen sich wie auch andere Marine Organismen durch spezielle dem Leben im Meer angepasste Inhaltsstoffe aus (Lindequist, U. and Th. Schweder: Marine Biotechnology, in: Biotechnology. 2. ed., Ed. H.-J. Rehm Wiley-VCH Weinheim 2001, pp. 442-473). Das gilt auch für die Pflanzenfamilie der Seegrasgewächse. Die Nutzung der Inhaltsstoffe wurde bei diversen Meerespflanzen für unterschiedliche Anwendungen beschrieben. Seegrasgewächse sind untergetaucht lebende Höhere Blütenpflanzen. Sie kommen in mehreren Arten überwiegend auf der Nordhälfte der Erde im Flachwasserbereich an den Küsten der Meere bis zu einer Tiefe von 12 m, vorwiegend in 4 m Wassertiefe vor. Neben der enormen Bedeutung der Seegraswiesen als Lebensstätte für Fische und andere Meeresorganismen dienen sie der Stabilisierung der Meeressedimente und der Ernährung bestimmter Meerestiere.

Seegras wird meist mit anderem organischen Material bei bestimmten Witterungsbedingungen als Strandgut angespült und steht daher als Rohstoffquelle kostengünstig zur Verfügung.

Schon vor Jahrhunderten sind weltweit Seegräser aufgrund ihrer außergewöhnlichen Eigenschaften als Isolier- und Füllstoff eingesetzt worden. Zu für diese Anwendung günstigen Eigenschaften zählen vor allem die gute schall- und wärmeisolierende Wirkung, eine gute Verrottungsfestigkeit und eine Schwerentflammbarkeit. Der Einsatz von Seegras der Gattung *Zostera* in Produkten für den Einsatz als Isolier- und Dämmmaterial sowie in Produkten gegen Ungeziefer und Wanzen wurde patentrechtlich geschützt (US4016084, CA1096193). Zum Stand der Technik gehört die Verwendung von pulverisierten Seegräsern in Körperpackungen (DE 20008368).

Beschrieben wurde ferner die Verwendung der Inhaltsstoffe von Seegras in der Lebensmitteltechnologie. So ist die Nutzung eines Pektins, erhältlich aus einem in Asien vorkommenden Seegras, Zostera *asiatica,* zur Herstellung von Erzeugnissen der Lebensmittelindustrie wie alkoholfreies Tonic (RU2125816, RU2128454), zur Käseherstellung (RU2128918) bekannt. Eine pharmazeutische Nutzung von Extrakten von Arten der Familie Zosteraceae wurde bisher nicht beschrieben.

**Aufgaben der Erfindung**

Aufgabe der vorliegenden Erfindung ist es, eine weitergehende Nutzung von Inhaltsstoffen der Familie Zosteraceae, insbesondere der Gattung *Zostera* zu ermöglichen, indem Extrakte dieser marinen Organismen auf kostengünstigem direkten Weg zu pharmazeutischen Zubereitungen für eine gezielte Prophylaxe oder Therapie entwickelt werden, die dem oben genannten Stand der Technik überlegen sind.

**Darlegung des Wesens der Erfindung**

Erfindungsgemäß sind für eine Verwendung in der Human- und Veterinärindustrie geeignete biologisch aktive Extrakte aus der Familie Zosteraceae durch Behandlung mit Lösungsmitteln erhältlich.

Die Extrakte sind vorteilhaft erhältlich aus den Spezies *Zostera marina* und/oder *Zostera noltii.* Damit wird eine bisher nicht verwirklichte technische Lösung zur arzneilichen Verwertung der wertvollen Inhaltsstoffe dieser Pflanzen erreicht.

Aus dem natürlich vorkommenden Seegras können mit Lösungsmitteln unterschiedlicher Polarität Extrakte hergestellt werden. Dabei können bekannte Extraktionsmittel wie kaltes und heißes Wasser, Methanol, Ethanol, Aceton, Ethylether, n-Hexan, Ethylazetat, eingesetzt werden, besonders vorteilhaft sind jedoch Lösungsmittel, die bei der Extraktion Seegras bisher nicht eingesetzt wurden wie Ethylacetat. Erfindungsgemäße Extrakte werden vorteilhaft erhalten durch eine Extraktion mit einem lipophilen Lösungsmittel. Erfindungsgemäße Extrakte werden ebenfalls erhalten durch eine Extraktion mit Ethylacetat. Extrakte, erhältlich durch eine Extraktion mit einwertigen Alkoholen, werden in besonders hohen Ausbeuten gewonnen.

Ebenso ist es erfindungsgemäß, Extrakte durch eine Wasserextraktion zu gewinnen, vorzugsweise bei Temperaturen von 10°C-80°C, wobei die Extraktion vorteilhaft stufenweise mit Wasser mit ansteigender Temperatur ausgeführt wird.

Es ist möglich, die Extraktion in mehreren Schritten mit Lösungsmitteln unterschiedlicher Polarität durchzuführen. Erfindungsgemäße Extrakte werden erhalten durch eine Extraktion des Rückstandes, der bei der Extraktion mit lipophilen Lösungsmitteln erhalten wird

Ethanolische und wässrige Extrakte aus Seegras sind stark antiviral wirksam. Ebenso wurde gefunden, daß Extrakte eine Wirksamkeit gegen grampositve Bakterien aufweisen. Es war nicht vorhersehbar, dass Pflanzen-Extrakte erhältlich sind, die in der Lage sind, Viren und multiresistente Bakterien zu hemmen und deshalb für spezielle prophylaktische und therapeutische Anwendungen in der Human- und Veterinärmedizin genutzt werden können. Die Extrakte können bei verschiedenen Anwendungsgebieten direkt eingesetzt werden. Es ist ebenso erfindungsgemäß, die Extrakte mit chromatographischen Verfahren weiter zu verarbeiten und aus diesen Gelen, Salben, Cremes, Tinkturen, Fluids, Hautmilch, Tabletten, Pulver, Kapseln und Ampullen zu formulieren.

Die Verwendung einer oder mehrere Extrakte gemäß Beispiel 1 und 2 ist bei verschiedenen Erkrankungen möglich. Insbesondere ist eine topische Anwendung zur Prophylaxe und/oder Therapie von Herpes-Erkrankungen vorteilhaft. Die Verwendung biologisch aktiver Extrakte als vitalisierende Zusatzstoffe (Cosmeceutical) bewirkt einerseits auf Grund der nachgewiesenen antiviralen Wirkung einen therapeutischen und/oder prophylaktischen Effekt und nutzt darüber hinaus die Kombination günstiger Eigenschaften zur Linderung der mit der Herpes-Infektion verbundenen Schmerzen.

Die Verwendung biologisch aktiver Extrakte aus der Familie Zosteraceae löst Probleme, die in jüngster Zeit der Human- und Veterinärmedizin bei der Bekämpfung multiresistenter Bakterien aufgetreten sind. Die Erweiterung der Palette antimikrobiell wirksamer Substanzen ist sehr wichtig, um für die Human- und Veterinärmedizin Mittel mit antimikrobieller Wirkung und unterschiedlichen Anwendungseigenschaften zur Verfügung zu stellen. Dies gilt insbesondere für Mittel mit Wirksamkeit auch bei multiresistenten grampositiven Keimen.

In der Veterinärmedizin spielen Staphylokokkeninfektionen vor allem in der Rinderhaltung eine große Rolle, da die Keime beim Melken leicht übertragen werden und die Milch kontaminieren können. Die Anwendung der Extrakte und/ oder von Zubereitungen mit diesen Extrakten ist lokal möglich. Durch Verwendung der Extrakte nach Anspruch 1, 2, 4, 5 und 7 in Zubereitungen für Anwendungen am Euter kann eine Übertragung von Staphylokokkeninfektionen verhindert und eine Kontamination der Milch vermieden werden.

Auf den nachgewiesen biologischen Wirkungen basiert die erfindungsgemäße Anwendung als Arzneimittel, die einen oder mehrere Extrakte enthalten. Zubereitungen, enthaltend eine oder mehrere der Extrakte, können insbesondere zur Herstellung eines Arzneimittels zur zielgerichteten Prophylaxe und Behandlung von grampositiven Infektionen sowie von Virusinfektionen vorteilhaft sein.

Die Erfindung soll nachstehend an einigen Beispielen erläutert werden, ohne sich auf diese Beispiele zu beschränken.

Ausführungsbeispiele

**Beispiel 1**

Herstellung des n-Hexan-Extraktes

Das geschnittene bzw- pulverisierte Material von *Zostera* wurde in Extraktionshülsen eingefüllt und mit der 30fachen Menge Hexan (m/V) je 3 x 8 h extrahiert. Das Lösungsmittel wurde im Vakuum entfernt. Die Ausbeute an Trockenextrakt betrug 0,6% bzw. 0,8%.

Herstellung des Ethylazetat-Extraktes

Der nach der Hexanextraktion in der Extraktionshülse verbleibende Rückstand des geschnittenen bzw. pulverisierten Materials von *Zostera* wurde an der Luft getrocknet und anschließend mit der 30fachen Menge Ethylazetat jeweils 3 x 8 h extrahiert. Das Lösungsmittel wurde im Vakuum entfernt. Die Ausbeute an Trockenextrakt betrug 0,3%.

Herstellung des Methanolextrakts

Der nach der Herstellung des Ethylazetatextrakts in der Extraktionshüle verbleibende Rückstand des geschnittenen bzw. pulverisierten Materials von *Zostera* wurde an der Luft getrocknet und anschließend mit der 30fache Menge Methanol jeweils 3 x 8 h extrahiert. Das Lösungsmittel wurde im Vakuum entfernt. Die Ausbeute an Trockenextrakt betrug 1,6%.

Herstellung des Wasserextrakts

Der nach der Herstellung des Methanolextrakts in der Extraktionshüle verbleibende Rückstand des geschnittenen bzw. pulverisierten Materials von *Zostera* wurde an der Luft getrocknet. Nach Überführen in einen Erlenmeyerkolben wurde das Material mit der 30fache Menge Wasser jeweils 3 x 3 h extrahiert. Der Extrakt wurde im Vakuum aufkonzentriert und anschließend durch Lyophilisation getrocknet. Die Ausbeute an Trockenextrakt betrug 4%.

**Beispiel 2**

Anwendung bei Herpesvirus-Infektionen

Die zu testenden Extrakte bzw. Substanzen wurden im Neutralrotfreisetzungstest auf antivirale Aktivität geprüft.

Methodik des Neutralisationsfreisetzungstestes

Vero-Zellen wurden in 96er Zellkulturplatten in Zellkulturmedium (MEM mit Zusatz von 5% FCS) in einer Konzentration von 400000 Zellen/ml ausgesät und für 24 Stunden bei 37°C inkubiert. Nach Präinkubation der Zellen mit 100µl der entsprechenden Substanzkonzentration (100; 50; 25; 12,5 µg/ml in Vierfachbestimmungen) für 30 min. bei 37°C erfolgte die Infektion mit 30 $TCID_{50}$ Herpes simplex-Virus Typ 1 (HSV-1, 50 µl/Vertiefung). Die Kulturen wurden für weitere 72 Stunden bei 37°C inkubiert. Nach Entfernen des Zellkulturüberstandes wurden 200 µl einer Neutralrotlösung (0,005%) in MEM hinzugefügt und 3 Stunden bei 37°C inkubiert. Der durch die lebenden Zellen inkorporierte Farbstoff wurde dann durch Elution mit 100 µl eines Ethanol/Wasser/Essigsäure-Gemisches (50:50:1) unter Schütteln für 15 min freigesetzt. Die Messung der Extinktion erfolgte bei 540 nm und die Berechnung des Schutzeffektes nach der Formel:

$$[(OD_t)_{Virus} - (OD_c)_{Virus} / (OD_c)_{mock} - (OD_c)_{Virus}] \times 100\ (\%)$$

t: mit Substanz, c: ohne Substanz

Ergebnisse

Tabelle 1:

| Testung auf antivirale Effekte gegen *Herpes simplex*-Virus (HSV1) | | | | |
|---|---|---|---|---|
| | Extrakt | Konzentration µg/ml | Schutz gegen HSV1 % | |
| | | | Versuch 1 | Versuch 2 |
| **Standort 1** | wäßrig Soxhlet | 100 | 100 | 100 |
| | | 50 | 100 | 100 |
| | | 25 | 100 | 100 |
| | | 12,5 | 0 | 8,2 |
| | wäßrig Rühren | 100 | 100 | 100 |
| | | 50 | 100 | 100 |
| | | 25 | 25 | 68 |
| | | 12,5 | 0 | 2,5 |
| **Standort 3** | wäßrig Soxhlet | 100 | 100 | 100 |
| | | 50 | 100 | 100 |
| | | 25 | 0 | 0 |
| | | 12,5 | 0 | 0 |
| | wäßrig Rühren | 100 | 100 | 100 |
| | | 50 | 43 | 64 |
| | | 25 | 0 | 0 |
| | | 12,5 | 0 | 0 |

**Beispiel 3**

Verhinderung von Staphylokokkenübertragungen durch Vorbehandlung der Euter Testung auf sensible und multiresistente Staphylokokken

Die zu prüfenden Extrakte wurden im Agardiffusionstest auf bakteriostatische Wirksamkeit geprüft.

Methodik des Agardiffusionstestes:

Die zu testenden Extrakte bzw. Substanzen wurden jeweils mit entsprechenden Lösungsmitteln (Dichlormethan, Methanol und Wasser) aufgelöst und auf Filterpapierplättchen in verschiedenen Konzentrationen aufgetragen. Zur Kontrolle dienten Vergleichsplättchen, die mit 25 µl des entsprechenden Lösungsmittels durchtränkt wurden. Als Referenzsubstanz wurde Ampicillin (10 µg/Plättchen) eingesetzt. Die getrockneten Filterpapierplättchen wurden abschließend auf die fertig hergestellten Agarplatten aufgelegt. Für die Fertigstellung der Agarplatten wurde mit einer Impföse eine stecknadelkopfgroße Menge an Testkeimkultur in 3 ml einer sterilen 0,9%igen NaCI-Lösung suspendiert. Anschließend wurden 200 µl der Keimsuspension zu 20 ml sterilen, verflüssigten und wenig warmen Nähragar zugesetzt und in Petrischalen mit einem Durchmesser von 10 cm ausgegossen. Nach Auflegung der Plättchen auf dem festen Agar wurden die Platten für ca. 2 bis 3 Stunden bei 8 °C vorinkubiert. Danach erfolgte eine Inkubation der Platten im Inkubationsschrank bei 37 °C für 24 Stunden. *Micrococcus flavus* bildete eine Ausnahme. Diese Kultur wurde nur bei Raumtemperatur gelagert, da der Keim sein Wachstumsoptimum bei 20 °C erreicht. Die Auswertung der Platten erfolgte durch Ausmessen der Durchmesser der Hemmhöfe. Sämtliche Arbeiten erfolgten unter aseptischen Bedingungen.

**Ergebnisse:**

Tabelle 2:

| Antibakterielle Testung von Extrakten aus Seegras von 3 Standorten | | | | | | | |
|---|---|---|---|---|---|---|---|
| Wenn nicht anders angegeben 2 mg Seegras/Plättchen<br>( ) Hemmung des Bakterienwachstums auf der Oberfläche, in Tiefe Bakterien vorhanden o.E. ohne Effekt<br>Angabe der Hemmhofbreite = Hemmhofdurchmesser- Plättchendurchmesser: 2<br>Agarplattendiffusionstest/Plättchenmeth./4 h Vordiffusion bei Raumtemp./24 h Inkubation bei 37°C | | | | | | | |
| **Standor t** | **Extrakt** | ***Bacillus subtilis*** | | ***Staphylococcus aureus*** | | ***Proteus mirabilis*** | |
| 1 | n-Hexan | | | | | | |
| | Soxhlet | (1 mm) | (0,5mm) | 0,5 mm | 0,5 mm | o.E. | o.E. |
| | Rühren | (2 mm) | 2mm | 3 mm | 6 mm | o.E. | o.E. |
| | Wasser | | | | | | |
| | Soxhlet | o.E. | o.E. | 4 mm | 3 mm | 3 mm | 2 mm |
| | Rühren | o.E. | o,E. | o.E. | o.E. | o.E. | o.E. |
| 2 | n-Hexan | | | | | | |
| | Soxhlet | (1mm) | (0,5 mm) | o.E. | o.E. | o.E | o.E |
| | Rühren | (1 mm) | 1 mm | o.E. | o.E. | o.E | o.E.. |
| 3 | n-Hexan | | | | | | |
| | Soxhlet | (2mm) | 1 mm | 0,5 mm | 0,5 mm | o.E. | o.E. |
| | Rühren | (3mm) | 4 mm | 1 mm | 6 mm | o.E. | o.E. |

Tabelle 3:

| **Effekte gegen multiresistente Staphylokokken des wässrigen Extrakts aus *Zostera* Hemmhofdurchmesser in mm** | |
|---|---|
| | Standort 1 |
| Wässriger Extrakt | |
| *Staphylococcus aureus* ATTC | 12 |
| S. *aureus* Norddeutscher Stamm | 16 |
| S. *aureus* | 16 |

**Patentansprüche**

1.  Verwendung biologisch aktiver Extrakte, erhältlich aus der Pflanzenfamilie Zosteraceae, durch Behandlung mit Lösungsmitteln zur topischen und systemischen Anwendung in der Humanmedizin und/oder in der Veterinärmedizin.

2.  Verwendung biologisch aktiver Extrakte, erhältlich aus *Zostera marina* und/oder *Zostera noltii* durch Behandlung mit Lösungsmitteln zur topischen und systemischen Anwendung in der Humanmedizin und/oder in der Veterinärmedizin.

3.  Verwendung der Extrakte nach Anspruch 1 und 2 zur Vorbeugung und Therapie viraler Erkrankungen.

4.  Verwendung der Extrakte nach Anspruch 1 und 2 zur Vorbeugung und Therapie bakterieller Infektionen.

5.  Verwendung der Extrakte nach Anspruch 1 und 2 in der Veterinärmedizin zur Verhinderung der Übertragung von Staphylokokkeninfektionen in der Rinderhaltung, **dadurch gekennzeichnet, dass** die Extrakte in Zubereitungen für Anwendungen am Euter eingearbeitet werden.

6.  Zubereitungen, enthaltend eine oder mehrere der Extrakte nach Anspruch 1 und 2, in Form von Gelen, Salben, Cremes, Tinkturen, Fluids, Hautmilch, Tabletten, Pulvern, Kapseln und Ampullen zur Prophylaxe und Behandlung von Virusinfektionen.

7.  Zubereitungen, enthaltend eine oder mehrere der Extrakte nach Anspruch 1 und 2, in Form von Gelen, Salben, Cremes, Tinkturen, Fluids, Hautmilch, Tabletten, Pulvern, Kapseln und Ampullen zur Prophylaxe und Behandlung von grampositiven Infektionen.

8.  Verwendung der Extrakte nach Anspruch 1 und 2 zur Verwendung als Mittel zur Desinfektion.

Europäisches
Patentamt

**EUROPÄISCHER RECHERCHENBERICHT**

Nummer der Anmeldung

EP 03 00 3788

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 00 16764 A (ALBERTE RANDALL S ;ZIMMERMAN RICHARD C (US); PHYCOGEN INC (US)) 30. März 2000 (2000-03-30) <br> * Seite 4, Zeile 15 * <br> * Seite 14, Zeile 28 * <br> * Seite 11, Zeile 3 - Zeile 5 * <br> * Seite 11, Zeile 32 * <br> --- | 1-8 | A61K35/78 <br> A61L2/00 <br> A61P31/12 <br> A61P31/04 |
| X | DE 200 08 368 U (DOETSCH JUTSCH CHRISTEL) 7. September 2000 (2000-09-07) <br> * Seite 2, Absatz 6 * <br> --- | 1-4,6,7 | |
| X | DATABASE WPI <br> Section Ch, Week 199402 <br> Derwent Publications Ltd., London, GB; <br> Class B04, AN 1994-014514 <br> XP002240706 <br> & SU 1 782 601 A (AS USSR FAR E PACIFIC OCEAN BIOORG INST), <br> 23. Dezember 1992 (1992-12-23) <br> * Zusammenfassung * <br> --- | 1,2,6,7 | |
| | | | **RECHERCHIERTE SACHGEBIETE (Int.Cl.7)** |
| X | APOROZHETS T S ET AL: "ANTIBACTERIAL AND THERAPEUTIC EFFICACY OF PECTIN FROM ZOSTERA SEAGRASS" <br> ANTIBIOTIKI I KHIMIOTERAPIYA, <br> Bd. 36, Nr. 4, 1991, Seiten 24-26, <br> XP009010392 <br> ISSN: 0235-2990 <br> * Seite 26, rechte Spalte * <br> --- | 1,2,4-8 | A61K <br> A61L <br> A61P |

-/--

Der vorliegende Recherchenbericht wurde für alle Patentansprüche erstellt

| Recherchenort | Abschlußdatum der Recherche | Prüfer |
|---|---|---|
| MÜNCHEN | 12. Mai 2003 | Thalmair, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**Europäisches Patentamt**

# EUROPÄISCHER RECHERCHENBERICHT

Nummer der Anmeldung

EP 03 00 3788

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | HARRISON P G ET AL: "INHIBITION OF THE GROWTH OF MICRO ALGAE AND BACTERIA BY EXTRACTS OF EELGRASS ZOSTERA-MARINA LEAVES" MARINE BIOLOGY (BERLIN), Bd. 61, Nr. 1, 1980, Seiten 21-26, XP001147478 ISSN: 0025-3162 * Seite 25, linke Spalte * * Seite 22, linke Spalte, Absatz 2 * --- | 1-8 | |
| X | US 5 906 825 A (CRAVER III WILLIAM E ET AL) 25. Mai 1999 (1999-05-25) * Spalte 10, Zeile 20 * * Spalte 14, Zeile 12 * --- | 1-8 | |
| A | PATENT ABSTRACTS OF JAPAN vol. 016, no. 411 (C-0979), 31. August 1992 (1992-08-31) & JP 04 139108 A (KIYOUEI KAGAKU KOUGIYOU KK), 13. Mai 1992 (1992-05-13) * Zusammenfassung * ----- | 1-8 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| MÜNCHEN | 12. Mai 2003 | Thalmair, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

EP 1 338 286 A1

**ANHANG ZUM EUROPÄISCHEN RECHERCHENBERICHT**
**ÜBER DIE EUROPÄISCHE PATENTANMELDUNG NR.**　　　EP 03 00 3788

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-05-2003

| Im Recherchenbericht angeführtes Patentdokument | Datum der Veröffentlichung | Mitglied(er) der Patentfamilie | Datum der Veröffentlichung |
|---|---|---|---|
| WO 0016764　A | 30-03-2000 | AU　1094300 A | 10-04-2000 |
| | | AU　6261499 A | 10-04-2000 |
| | | AU　6264099 A | 10-04-2000 |
| | | AU　6264399 A | 10-04-2000 |
| | | BR　9913912 A | 19-06-2001 |
| | | BR　9914036 A | 12-06-2001 |
| | | BR　9914498 A | 26-06-2001 |
| | | BR　9914499 A | 26-06-2001 |
| | | CA　2345111 A1 | 30-03-2000 |
| | | CA　2345231 A1 | 30-03-2000 |
| | | CA　2345232 A1 | 30-03-2000 |
| | | CA　2345233 A1 | 30-03-2000 |
| | | EP　1115284 A1 | 18-07-2001 |
| | | EP　1115289 A2 | 18-07-2001 |
| | | EP　1115285 A1 | 18-07-2001 |
| | | EP　1115394 A2 | 18-07-2001 |
| | | JP 2002526427 T | 20-08-2002 |
| | | JP 2002526184 T | 20-08-2002 |
| | | JP 2002526053 T | 20-08-2002 |
| | | JP 2002526444 T | 20-08-2002 |
| | | WO　0016764 A2 | 30-03-2000 |
| | | WO　0016623 A1 | 30-03-2000 |
| | | WO　0016632 A2 | 30-03-2000 |
| | | WO　0016624 A1 | 30-03-2000 |
| | | US 2001051274 A1 | 13-12-2001 |
| DE 20008368　U | 07-09-2000 | DE　20008368 U1 | 07-09-2000 |
| SU 1782601　A | 23-12-1992 | SU　1782601 A1 | 23-12-1992 |
| US 5906825　A | 25-05-1999 | AU　1105199 A | 10-05-1999 |
| | | CA　2308588 A1 | 29-04-1999 |
| | | EP　1024796 A1 | 09-08-2000 |
| | | WO　9920258 A1 | 29-04-1999 |
| JP 04139108　A | 13-05-1992 | JP　2971549 B2 | 08-11-1999 |

Für nähere Einzelheiten zu diesem Anhang : siehe Amtsblatt des Europäischen Patentamts, Nr.12/82